# EUROPEAN PATENT SPECIFICATION

(11) **EP 4 305 637 B1**
(45) Date of publication and mention of the grant of the patent: **28.01.2026**
(21) Application number: 22709524.7
(22) Date of filing: 01.03.2022
(51) Int. Cl.: G16H 20/40, G16H 40/63, A61M 1/14, A61M 1/28

(54) **DIALYSIS SYSTEM HAVING A DIRECTIONAL ALARM SYSTEM**
DIALYSESYSTEM MIT EINEM RICHTUNGSALARMSYSTEM
SYSTÈME DE DIALYSE AVEC UN SYSTÈME D'ALARME DIRECTIONNEL

(30) Priority: 10.03.2021 US 202117197554
(43) Date of publication of application: 17.01.2024
(73) Proprietor: Fresenius Medical Care Holdings, Inc., Waltham, MA 02451-1457 (US); Fresenius Medical Care Deutschland GmbH, 61352 Bad Homburg (DE)
(72) Inventor: YUDS, David, Waltham, Massachusetts 02451-1457 (US); MOISSL, Ulrich, 61352 Bad Homburg (DE)
(74) Representative: Fish & Richardson P.C.
(86) International application number: PCT/US2022/018264
(87) International publication number: WO 2022/192029

(56) References cited:
- WO-A1-2020/257065
- JP-A- 2015 144 722
- US-A1- 2007 104 334
- US-A1- 2010 088 820

## Description

### TECHNICAL FIELD

This application relates generally to home dialysis systems having focused directional alarms including a directional speaker.

### BACKGROUND

Medical devices, such as dialysis machines, are known for use in the treatment of renal disease. The two principal dialysis methods are hemodialysis (HD) and peritoneal dialysis (PD). During hemodialysis, the patient's blood is passed through a dialyzer of a hemodialysis machine while also passing dialysate through the dialyzer. A semi-permeable membrane in the dialyzer separates the blood from the dialysate within the dialyzer and allows diffusion and osmosis exchanges to take place between the dialysate and the blood stream. During peritoneal dialysis, the patient's peritoneal cavity is periodically infused with dialysate, or dialysis solution. The membranous lining of the patient's peritoneum acts as a natural semi-permeable membrane that allows diffusion and osmosis exchanges to take place between the solution and the blood stream. Automated peritoneal dialysis machines, also called PD cyclers, are designed to control the entire peritoneal dialysis process so that it can be performed at home, usually overnight, without clinical staff in attendance. Both HD and PD machines may include displays with touch screens or other user interfaces that display information of a dialysis treatment and/or enable an operator or patient to interact with the machine.

Dialysis machines are equipped to generate alerts and/or alarms (hereinafter used interchangeably), include both audible and visual alarms, in response to conditions and situations requiring user attention. Many times, particularly during PD treatment using a PD cycler, alarms occur in the middle of the night when the patient is asleep and cause disturbance of the patient's sleep. Depending on the proximity to other people, the PD cycler can also disturb other people, like a spouse or caregiver in the same room, people in an adjacent bedroom, or even neighbors who share a common apartment wall. This creates frustration in other people who are not dialysis patients, and the stress of upsetting other people increases anxiety for the patient. Furthermore, the line of sight for dealing with a PD cycler alarm is usually such that the patient must sit up or get out of bed to look at the device's display screen, which may cause further disturbance to the patient.

Accordingly, it would be desirable to provide a system that addresses the above-noted concerns and other issues.

US 2010/0088820 describes use of a directional sound source, a medical treatment station and a treatment room, in which each such medical treatment station has a directional sound source assigned to it.

WO 2020/257065 describes a dialysis machine that can include a patient line that provides dialysate solution to a patient and removes effluent dialysate from the patient through a catheter. During a drain phase of a PD treatment, an occlusion can occur at different locations in the patient line and/or catheter. A pressure sensor can detect a change in pressure of the fluid at the proximal end of the patient line to infer a potential occlusion in the patient line.

JP 2015144722 describes a dialyzer that comprises a dialyzer body and a parametric loudspeaker electrically connected to the dialyzer body. The dialyzer body comprises a control part that is electrically connected to the parametric loudspeaker to control an orientation direction of the parametric loudspeaker and an audible sound generation position.

### SUMMARY

The invention is as set forth in the appended claims.

A home dialysis system is defined in claim 1.

According further to the system described herein, a non-transitory computer readable medium has executable code that when executed by a processor causes execution of a method for handling notification of a dialysis alarm by a home dialysis system is defined in claim 12.

According to aspects of the system described herein, multiple feature implementations may be provided in connection with the dialysis system, method of handling alarm notifications, and non-transitory computer readable medium described herein. The directional alarm system may comprise an orientation mechanism to orient the emitting of the directional sound waves in the controlled direction that may be a manual mechanism and/or an automated mechanism that includes a sensor used to locate automatically a desired direction for emitting the directional sound waves. In response to the generation of the alarm, a speaker of the dialysis machine may be deactivated for a period of time while the directional alarm system is functioning. The directional alarm system may include a display component that provides visual display of an alarm. The directional alarm system may include a receiving device disposed remotely from the dialysis machine that receives the directional sound waves and directs the directional sound waves towards a fixed location. The directional alarm system may be in wireless communication with the dialysis machine. If the alarm generated by the alarm generation component is not addressed for a specified time, a safety protocol may be initiated that includes stopping emission of the directional sound waves and causing audible general broadcast of the alarm. A volume of the alarm conveyed by the directional sound waves may be configured to increase automatically from a quieter volume to a louder volume.

### BRIEF DESCRIPTION OF THE DRAWINGS

Implementations and features of the system described herein are explained with reference to the several figures of the drawings, which are briefly described as follows.
FIG. 1A illustrates an exemplary implementation of a dialysis machine in a dialysis system configured for use in accordance with the present disclosure.
FIG. 1B is a schematic illustration of an exemplary embodiment of the dialysis machine that is configured for use in accordance with the present disclosure.
FIG. 2 illustrates another exemplary implementation of a dialysis machine that is configured for use in accordance with the present disclosure.
FIG. 3 is a schematic illustration showing a patient undergoing an overnight treatment by a dialysis system that includes a directional speaker system according to an implementation of the system described herein.
FIG. 4 is a schematic illustration showing another implementation of a directional alarm system that may include directional speakers integrated in connection with a display panel.
FIG. 5 is a schematic illustration showing another implementation of the system described herein to direct the focused sound wave coming from a directional speaker system by using a receiving device on the ceiling above the bed of the patient.
FIG. 6 is a schematic illustration showing other implementations of the system described herein for providing alarms, including directional alarms and visual displays.
FIG. 7 is a flow diagram showing an example implementation of alarm notification handling by a dialysis system having a directional alarm system according to the system described herein.

### DETAILED DESCRIPTION

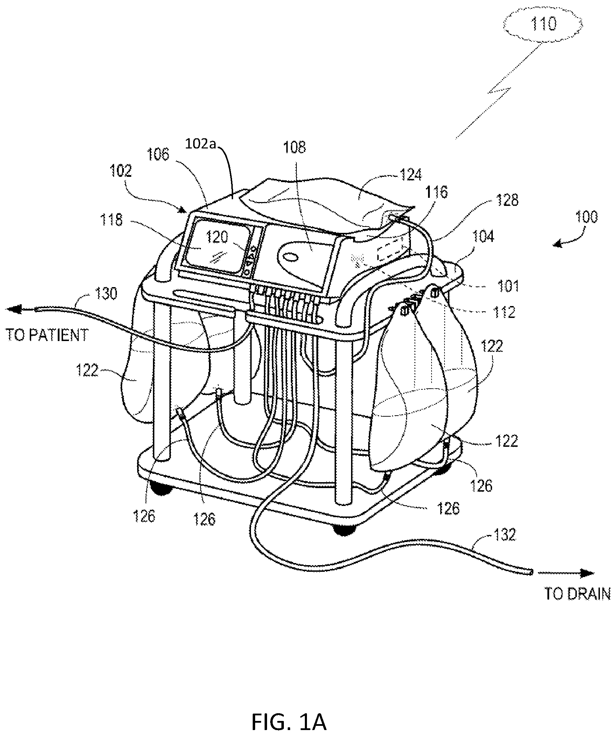

FIG. 1A shows an example of a medical device, implemented as a peritoneal dialysis (PD) system 100, that is configured for use in accordance with an exemplary implementation of the system described herein. The PD system 100 configured for use at a patient's home (i.e., it is a home dialysis system). The PD system 100 may include a dialysis machine 102 (e.g. a PD machine, also referred to as a PD cycler) which in some embodiments may be seated on a cart 104. The dialysis machine 102 may include a housing 106, a door 108, and a cartridge interface for contacting a disposable PD cassette, or cartridge, when the cartridge is disposed within a compartment formed between the cartridge interface and the closed door 108. A heater tray 116 may be positioned on top 102a of the housing 106. The heater tray 116 may be any size and shape to accommodate a bag of dialysate (e.g., a 5L bag of dialysate). The dialysis machine 102 may also include a user interface such as a touch screen 118 and control panel 120 operable by a user (e.g., a caregiver or a patient) to allow, for example, set up, initiation, and/or termination of a PD treatment.

Dialysate bags 122 may be suspended from the sides of the cart 104, and a heater bag 124 may be positioned in the heater tray 116. Hanging the dialysate bags 122 may improve air management as any air is disposed by gravity to a top portion of the dialysate bag 122. Valves may be attached to a bottom portion of the dialysate bags 122 so fluid is drawn out and air delivery is minimized. Dialysate from the dialysate bags 122 may be transferred to the heater bag 124 in batches. For example, a batch of dialysate may be transferred from the dialysate bags 122 to the heater bag 124, where the dialysate is heated by the heating element. When the batch of dialysate has reached a predetermined temperature (e.g., approximately 98°-100°F, 37°C), the batch of dialysate may be flowed into the patient. The dialysate bags 122 and the heater bag 124 may be connected to the cartridge via dialysate bag lines 126 and a heater bag line 128, respectively. The dialysate bag lines 126 may be used to pass dialysate from dialysate bags 122 to the cartridge during use, and the heater bag line 128 may be used to pass dialysate back and forth between the cartridge and the heater bag 124 during use. In addition, a patient line 130 and a drain line 132 may be connected to the cartridge. The patient line 130 may be connected to a patient's abdomen via a catheter and may be used to pass dialysate back and forth between the cartridge and the patient's peritoneal cavity during use. The drain line 132 may be connected to a drain or drain receptacle and may be used to pass dialysate from the cartridge to the drain or drain receptacle during use.

The touch screen 118 and the control panel 120 may allow a user to input various treatment parameters to the dialysis machine 102 and to otherwise control the dialysis machine 102. In addition, the touch screen 118 may serve as a display. The touch screen 118 may function to provide information to the patient and the operator of the PD system 100. For example, the touch screen 118 may display information related to a dialysis treatment to be applied to the patient, including information related to a prescription. In various embodiments, the control panel 120 may also include audio and video component capabilities, including speakers, microphones and/or cameras.

The dialysis machine 102 may include a processing module 101 that resides inside the dialysis machine 102, the processing module 101 being configured to communicate with the touch screen 118 and the control panel 120. The processing module 101 may be configured to receive data from the touch screen 118 the control panel 120 and sensors, e.g., temperature and pressure sensors, and control the dialysis machine 102 based on the received data. For example, the processing module 101 may adjust the operating parameters of the dialysis machine 102.

The dialysis machine 102 may be configured to connect to a network 110. The connection to network 110 may be via a wireless connection, such as via WiFi or Bluetooth, or in some cases a non-wireless connection, as further discussed elsewhere herein. The dialysis machine 102 may include a connection component 112 configured to facilitate the connection to the network 110. The connection component 112 may be a transceiver for wireless connections and/or other signal processor for processing signals transmitted and received over a wired connection. In the case of a wired connection, the connection component 112 may be a port enabling a physical connection to a network component. Other medical devices (e.g., other dialysis machines) or components may be configured to connect to the network 110 and communicate with the dialysis machine 102. Although discussed herein principally in connection with a peritoneal dialysis machine, the system described herein may be used and implemented in connection with other types of medical devices having one or more displays, including home hemodialysis machines and/or other home medical devices.

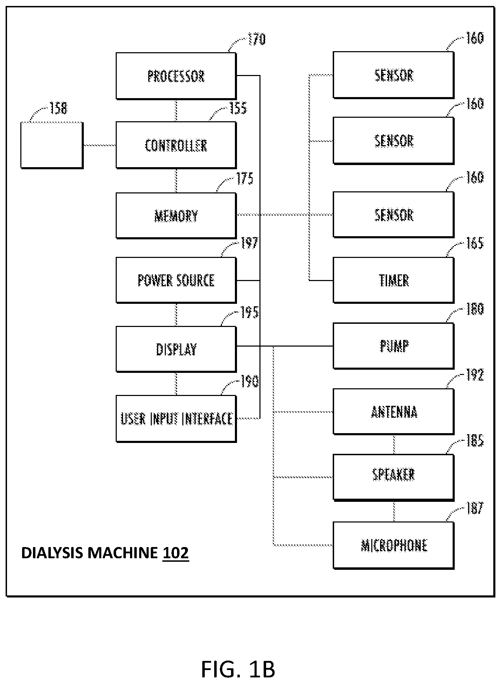

FIG. 1B is a schematic illustration of an exemplary embodiment of a dialysis machine such as, for example, the dialysis machine 102 that is configured for use in accordance with the present disclosure. The machine 102 may be a home dialysis machine, e.g., a PD machine, for performing a dialysis treatment on a patient, and may be included in the system 100 described above. A controller 155, that may be a component of the processing module 101, may automatically control execution of a treatment function during a course of dialysis treatment. The controller 155 may be operatively connected to the sensors 160 and deliver a signal to execute a treatment function or a course of treatment associated with various treatment systems. In some embodiments, a timer 165 may be included for timing triggering of the sensors 160.

In some embodiments, the machine 102 may also include a processor 170, and memory 175, the controller 155, the processor 170, and/or the memory 175, or combinations thereof, that may separately or collectively part of the processing module 101, that may receive signals from the sensor(s) 160 indicating various parameters. Each fluid bag (e.g., the dialysate bags 122) may contain an approximate amount of dialysate, such that "approximate amount" may be defined as a 3L fluid bag containing 3000 to 3150 mL, a 5L fluid bag containing 5000 to 5250 mL, and a 6L fluid bag containing 6000 to 6300 mL. The controller 155 may also detect connection of all fluid bags 122 connected.

Communication between the controller 155 and the treatment system may be bidirectional, whereby the treatment system acknowledges control signals, and/or may provide state information associated with the treatment system and/or requested operations. For example, system state information may include a state associated with specific operations to be executed by the treatment system (e.g., trigger pumps and/or compressors to deliver dialysate and the like) and a status associated with specific operations (e.g., ready to execute, executing, completed, successfully completed, queued for execution, waiting for control signal, and the like).

In some embodiments, the dialysis machine 102 may include at least one pump 180 operatively connected to the controller 155. During a treatment operation, the controller 155 may control the pump 180 for pumping fluid, e.g., fresh and spent dialysate, to and from a patient. For example, the pump 180 may transfer dialysate from the dialysate bag 122 through, for example, a cassette insertable into a port formed in the dialysis machine, to the heating chamber 152 prior to transferring the dialysis to the patient. In an embodiment, the pump 180 may be a peristaltic pump. The controller 155 may also be operatively connected to a speaker 185 and a microphone 187 disposed in the machine 102. A user input interface 190 may include a combination of hardware and software components that allow the controller 155 to communicate with an external entity, such as a patient or other user. These components may be configured to receive information from actions such as physical movement or gestures and verbal intonation. In some embodiments, the components of the user input interface 190 may provide information to external entities. Examples of the components that may be employed within the user input interface 190 include keypads, buttons, microphones, touch screens, gesture recognition devices, display screens, and speakers. The machine 102 may also be wirelessly connectable via an antenna 192 for remote communication that may be a part of the connection component 112. The machine 102 may also include a display 195 and a power source 197.

The sensors 160 may be included for monitoring parameters and may be operatively connected to at least the controller 155, the processor 170, and/or the memory 175, or combinations thereof. The processor 170 may be configured to execute an operating system, which may provide platform services to application software, e.g., for operating the dialysis machine 102. These platform services may include inter-process and network communication, file system management and standard database manipulation. One or more of many operating systems may be used, and examples are not limited to any particular operating system or operating system characteristic.

The memory 175 may include a computer readable and writeable nonvolatile data storage medium configured to store non-transitory instructions and data. In addition, the memory 175 may include a processor memory that stores data during operation of the processor 170. In some examples, the processor memory includes a relatively high performance, volatile, random access memory such as dynamic random-access memory (DRAM), static memory (SRAM), or synchronous DRAM. However, the processor memory may include any device for storing data, such as a non-volatile memory, with sufficient throughput and storage capacity to support the functions described herein. Further, examples are not limited to a particular memory, memory system, or data storage system.

The instructions stored on the memory 175 may include executable programs or other code that may be executed by the processor 170. The instructions may be persistently stored as encoded signals, and the instructions may cause the processor 170 to perform the functions described herein. The memory 175 may include information that is recorded, on or in, the medium, and this information may be processed by the processor 170 during execution of instructions. The memory 175 may also include, for example, specification of data records for user timing requirements, timing for treatment and/or operations, historic sensor information, and the like. The medium may, for example, be optical disk, magnetic disk or flash memory, among others, and may be permanently affixed to, or removable from, the controller 155.

The sensor(s) 160 may include a pressure sensor for monitoring fluid pressure of the machine 102, although the sensors 160 may also include any of a heart rate sensor, a respiration sensor, a temperature sensor, a weight sensor, an air sensor, a video sensor, a thermal imaging sensor, an electroencephalogram sensor, a motion sensor, an audio sensor, an accelerometer, a capacitance sensor, or any other suitable sensor. It is appreciated that the sensors 160 may include sensors with varying sampling rates, including wireless sensors.

The controller 155 may be disposed in the machine 102 or may be coupled to the machine 102 via a communication port or wireless communication links, shown schematically as communication element 158 that may be a part of the connection component 112. According to various examples, the communication element 158 may support a variety of one or more standards and protocols, examples of which include wireless and/or non-wireless communication, such as USB, Wi-Fi, TCP/IP, Ethernet, Bluetooth, among others. As a component disposed within the machine 102, the controller 155 may be operatively connected to any of the sensors 160, the pump 180, and the like. The controller 155 may communicate control signals or triggering voltages to the components of the machine 102. As discussed, exemplary embodiments of the controller 155 may include wireless communication interfaces. The controller 155 may detect remote devices to determine if any remote sensors are available to augment any sensor data being used to evaluate the patient.

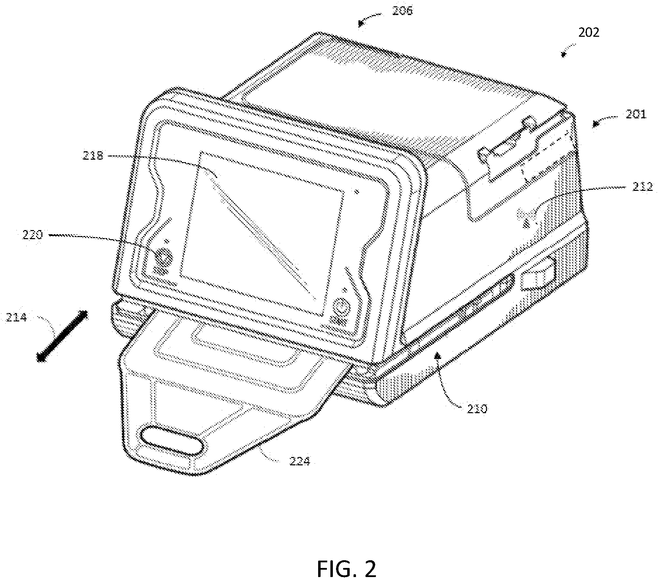

FIG. 2 is a schematic illustration showing another exemplary embodiment of a dialysis machine 202 in accordance with the present disclosure. The dialysis machine 202 may be implemented in the peritoneal dialysis system 100 and may have at least some similar components as that of the dialysis machine 102, for example, including a housing 206, a processing module 201, a connection component 212, a touch screen 218, and a control panel 220 operable by a user (e.g., a caregiver or a patient) to allow, for example, set up, initiation, and/or termination of a PD treatment. The processing module 201 and the connection component 212 may be configured similarly to the processing module 101 and connection component 112 described above. However, instead of a heater tray being positioned on a top surface 102a of the housing as shown in FIG. 1 for the dialysis machine 102, one or more heating elements may be disposed internal to the machine 202. For example, a warmer pouch 224 may be insertable into an opening 210 in a direction indicated at arrow 214. In embodiments, the warmer pouch 224 may be configured so dialysate may continually flow through the warmer pouch (instead of transferred in batches) to achieve a predetermined temperature before flowing into the patient.

The connection component 112, 212 may provide for connection of the dialysis machine 102, 202 through a secure gateway to connect to the network, including a network within the home and/or outside the home to send and receive information between devices and/or to a clinic. The connection, network and data transmissions among components, both local and external, may be controlled and/other otherwise incorporated into a system that facilitates such functions with appropriate network infrastructure, and which may, in some implementations, be referred to as a connected health system.

According to the system described herein, a medical device, such as a dialysis machine, may be equipped with focused directional speakers and/or displays to direct audible and visual alarms towards the patient, as an intended recipient, in order to minimize disruption to others caused by an alarm. Directional speakers are known that use ultrasonic technology to enable sound emission in a controlled and directional way. Examples of directional speakers include HyperSound speakers manufactured by Turtle Beach Corporation, and reference is made to HyperSound Hearing Solutions, White Paper: HyperSound Clear Technology, Turtle Beach Corporation, 2015. HyperSound speakers emit sound in a controlled, narrow beam by using ultrasound to create audio in the air. The ultrasonic carrier frequency is generated from the emitter at approximately 100 kHz. For further descriptions of directional sound systems, reference is made to US Patent Nos. 7,298,853 and 9,591,426.

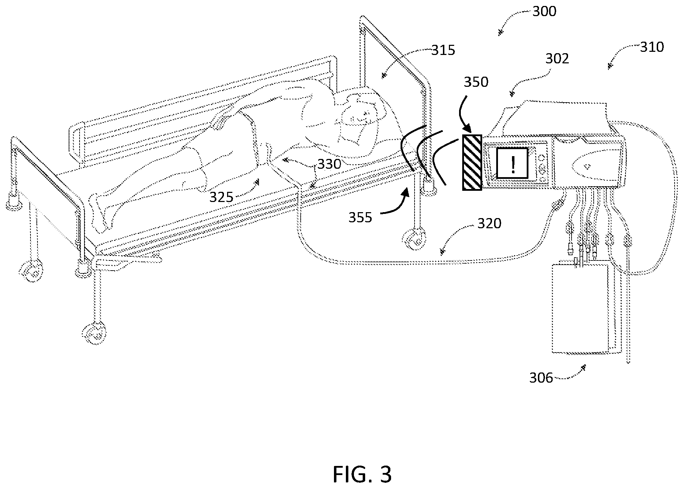

FIG. 3 is a schematic illustration 300 showing a patient undergoing an overnight treatment by a dialysis system 310 that includes a directional speaker system 350 according to an implementation of the system described herein. A dialysis machine, e.g. a PD cycler 302 that may have similar features one or more of the dialysis machines 102, 202 discussed elsewhere herein, may monitor the fluid flow to and from a patient 315. The patient 315, as illustrated, may be sleeping in a substantially horizontal position. It is also understood that the patient 315 may be sitting up and awake during treatment. Tubing 320, e.g., a patient fluid line, of the dialysis system 310 may extend between the PD cycler 302 and a catheter 325 extending from an abdomen (e.g., peritoneal cavity) of the patient 315. One or more dialysate bags 306 may be connected to the PD cycler 302, for providing fresh dialysate to the patient. The patient fluid line and the dialysate bags may be connected to each other and additional tubing via a cassette or cartridge. In embodiments, a patient line may be connected to the cartridge. The patient line may be connectable to the patient's abdomen (e.g., peritoneal cavity) via the catheter and the dialysis machine may be used to pass dialysate back and forth between the cartridge and the patient's peritoneal cavity during use with pump heads situated on the machine.

The PD cycler 302 includes software and hardware components to detect many types of errors, anomalies, inconsistencies, exceeding of set thresholds, etc. in connection with a dialysis treatment. As a result of detecting any such error, anomaly etc., a processing module of the PD cycler 302, like the processing module 110, that may include an alarm generation component, such as a processor executing alarm generation software, may perform alarm processing that includes generation of an audible alarm and/or display of an alarm message on the display screen of the PD cycler 302. For example, positioning of the tubing 320 during a treatment may be subject to how and where the patient may be sleeping and/or sitting. The tubing 320 may become kinked, or may collapse, or become otherwise blocked anywhere along the length of the tubing between the patient 315 and the PD cycler 302. Specifically, tubing 320 may kink, collapse, and/or block near the patient 315, e.g., at the catheter 325, and/or where the tubing extends beyond a patient's sleeping area, e.g., off an edge of a bed, or chair, indicated at reference numeral 330. The patient 315 may inadvertently kink, collapse, and/or otherwise block the tubing during normal shifting and/or movement while asleep. In response to detecting a potential kink, collapse, and/or blockage of the tubing 320, the PD cycler 302 may automatically generate an alert, alarm, and/or abort a treatment if fluid cannot freely flow between the patient 315 and the PD cycler 302. The PD cycler 302 may detect the kinked or collapsed tubing to provide warnings to the patient prior to stopping treatment, which are intended to wake the patient so that the tubing can be checked and readjusted as needed to continue the treatment. A kink alarm has been used as an example, but it should be clear that the system described herein may be used in connection with any appropriate alarm that may be generated before, during, or after a dialysis treatment.

According to an implementation of the system described herein, alarm processing and transmission may include causing deactivation of the normal speaker system of the PD cycler 302 for a period of time in favor of operation of the directional speaker system 350. In various implementations, the deactivation of the normal speaker system of the PD cycler 302 may be caused by a signal transmitted from the directional speaker system 350 to the PD cycler 302 and/or may be caused by the PD cycler 302 detecting that the directional speaker system 350 is coupled and functioning. As further described elsewhere herein, safety protocols may enable later actuation of the normal speaker system of the PD cycler 302 in specified circumstances. The directional speaker system 350 includes directional speakers, like that described above that emit directional sound waves, that may be either manually or automatically positioned to focus sound waves 355 directly on an intended recipient, such as the patient 315. In an implementation, as shown in the figure, the directional speaker system 350 may include an orientation mechanism, such as one or more hinge or joint mechanisms, to enable manual directional positioning of the sound wave emitter component of the speaker system 350. Various other implementations for positioning of the speakers to align with the patient 315 are described in further detail elsewhere herein. The speaker system 350 may be communicatively coupled to one or more processors of the dialysis machine 302, such as via a wired connection and/or via wireless connection, for example via WiFi and/or Bluetooth.

The speaker system 350 may be useful for home dialysis patients whose alarming devices at night wake up not only their partners but also neighbors, especially when the patient is hard of hearing or a heavy sleeper and needs to set the volume at the loudest setting in order to hear it to prevent possible serious injury. Accordingly, for example, in the kink alarm scenario discussed above, the dialysis machine 302 and/or other part of the dialysis system 310, may display an alarm (shown as a "! ") on the dialysis machine display and generate an audible alarm intended for the patient and activate the directional speaker system 350 to direct control and direction the sound waves 355 only to the patient so as to wake the patient, but minimize disturbance to others in the vicinity. The focused alert sound may be configured to progress from quiet to loud so as to not shock the patient awake. In further implemented safety features, a safety protocol may provide that, if the patient 315 still does not awaken after a set amount of time to clear the alarm, then the dialysis device generally broadcasts the alarm to the entire room.

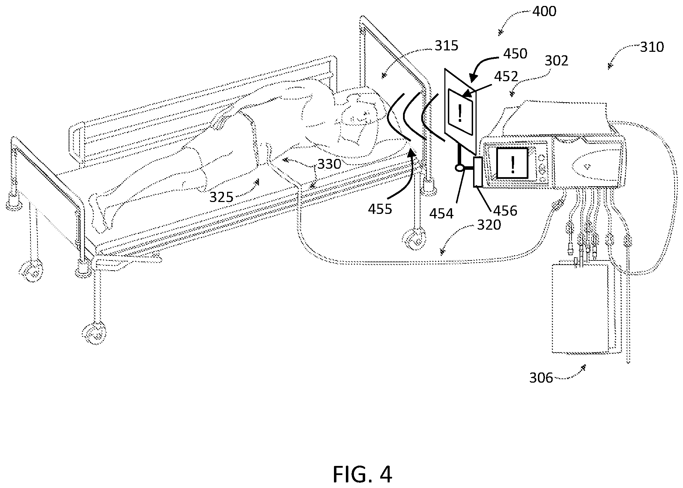

FIG. 4 is a schematic illustration 400 having corresponding elements as in the illustration 300 but further showing another implementation of a directional alarm system 450 that may include directional speakers integrated in connection with a display panel 452. The display panel 452 may be communicatively coupled to one or more processors of the dialysis machine 302, such as via a wired connection and/or via wireless connection, for example, via WiFi and/or Bluetooth. An implementation of the display panel 452 may include a sound-generating glass product that may be overlayed on the display panel 452 and which can be used to notify the patient of the dialysis machine status using focused directional sound that is generated from the sound-generating glass. In an implementation, the sound generating glass component of the display panel 452 may include a HyperSound Glass product that includes a glass pane layered with a set of transparent films allowing it to generate an ultrasound beam and includes appropriate processing components therefor.

Since the face of the sound generating glass of the display panel 452 is pointing directly at the patient, text of the visual alarm generated by the dialysis machine 302 and displayed on the display screen of the dialysis machine 302 may be transmitted or projected on the display panel 452. This provides that the patient can see it without needing to sit up or otherwise move to face the main screen of the dialysis machine 302, which may not be in a line of sight with the patient due to the room or treatment configuration. Additionally the display panel 452 may include controls that enable muting or clearing the alarm. These controls may be provided via touchscreen interfacing and/or remotely using infrared/optical/voice/etc. sensors so the patient does not need to get out of bed to interact with the dialysis machine user interface. The directional alarm system 450 may be connected to an arm 454 that may be positioned so that the display panel 452 is pointing at the patient's sleeping location thereby providing for the directional speaker functionality of the directional alarm system 450 to be focused and directed to the patient along with the visual display component. The arm 454 may be manually adjustable. In other implementations, a more advanced mechanism 456 may be provided that include sensors to automatically track the patient, for example, detecting the patient using laser/audio/infrared/etc. signals, and using those signals to control motors and actuators to align directionally the display panel 452 in a configuration to direct the directional speakers towards the patient 315.

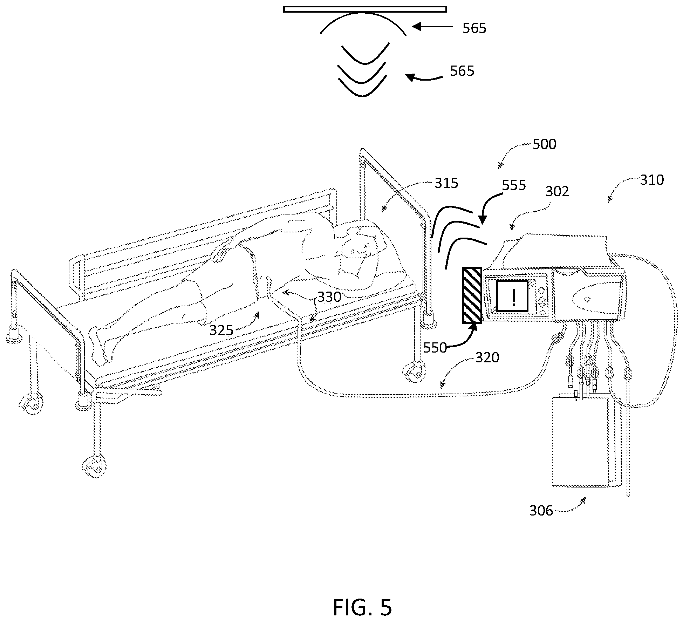

FIG. 5 is a schematic illustration 500 showing another implementation to direct the focused sound wave 555 coming from a directional speaker system 550 by using a receiving device 560 on the ceiling above the bed of the patient 315. Although shown on the ceiling above the patient, other remote locations for the receiving device may be used and provided for in connection with the system described herein. In various implementations, the receiving device 560 may either bounce (with a hyperbolic dish) and/or retransmit via another ceiling-mounted directional speaker the sound signal 565 down to the patient 315. This has the advantage of focusing from one fixed location vertically to a patient such that patient alignment and/or tracking needs may be reduced. For example, alignment sensors and/or wireless communication between the directional speaker system 550 and the receiving device 560 may provide for automated alignment and/or coupling of these devices to direct the sound waves to the patient when lying in bed.

In other implementations, for a patient with some hearing loss who cannot hear certain frequencies as well as others, the system may allow optimization of alarms via selection of the frequency to which the patient is best able to respond. The system could further enable selection of frequencies in which the perception ratio between the patient and the spouse is optimal to alert the patient but not disturb the spouse. This could be reached by a hearing test in which both the patient and spouse select the frequencies best suited to both wake the patient and not disturb the spouse. As a safety protocol, during an actual alarm event, the dialysis device may cycle through the alarm tones in the order of most preferred to least preferred if the patient does not respond within the designated time. This system may include periodic hearing checks to ensure the optimum frequency is produced.

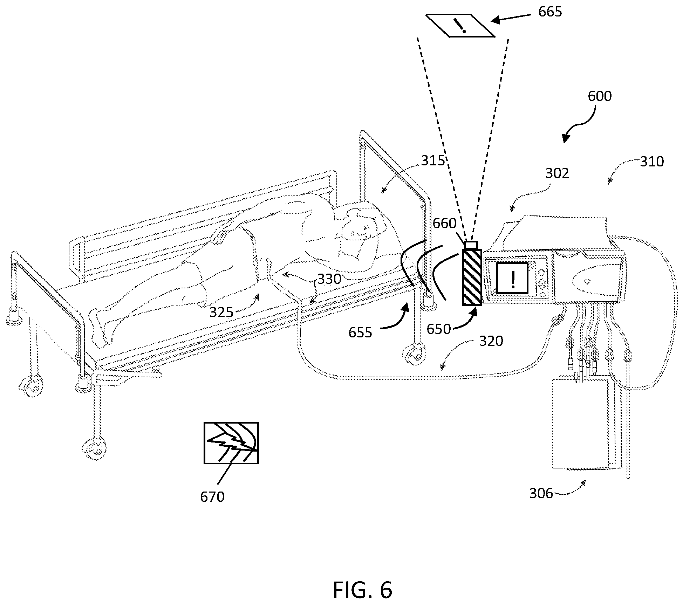

FIG. 6 is a schematic illustration 600 showing other implementations of the system described herein for providing alarms, including directional alarms and visual displays. To improve alarm cognition and viewing in the dark, the dialysis machine 302 and/or a directional alarm system 650, having features like the directional alarm features discussed elsewhere herein, may include a laser projected display 660 to project alarm messages 665 on the patient's wall or ceiling. This feature would be particularly helpful for far-sighted patients so they can immediately see the large alarm text at a distance when they would normally be fumbling for glasses to read a typical, small dialysis machine display screen. With some calibration, the alarm message could be made as large as the entire ceiling or wall for maximum effect. Additionally, to better ensure a quality night's rest for other people in a patient's room (home/assisted living/etc.), the speakers of the directional alarm system 650 may be paired with a noise-cancelling system 670 in a different part of the room that phase-shifts the alarm by 180 degrees to minimize alarm noise heard by others. Since the noise-cancelling system 670 may be programmed with the tone and frequency of the dialysis alarm, there would be no need for a microphone or processor found in traditional noise-cancelling systems.

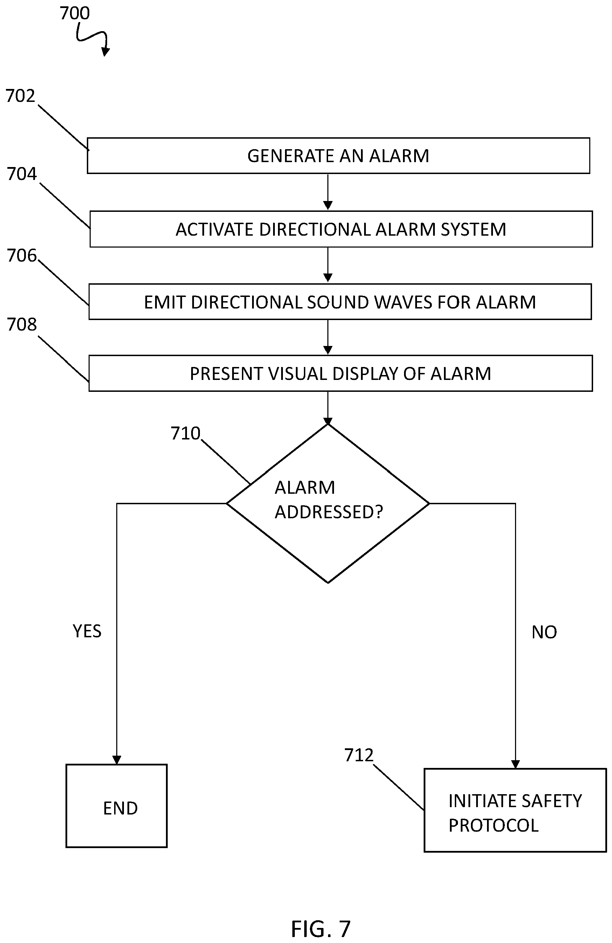

FIG. 7 is a flow diagram 700 showing an example implementation of an iteration of an alarm notification handling process by a dialysis system having a directional alarm system according to the system described herein. At a step 702, an alarm is generated at a dialysis machine using an alarm generation component, e.g. in response to an anomaly occurring during a dialysis treatment that requires user intervention. At a step 704, a directional alarm system of the dialysis machine is activated, wherein the directional alarm system includes a directional speaker that generates directional sound waves. Activating the directional alarm system may also include deactivating a speaker of the dialysis machine for a period of time while the directional alarm system is functioning. At a step 706, in response to the generation of an alarm by the alarm generation component of the dialysis machine, the directional sound waves indicating the alarm are emitted from the directional speaker in a controlled direction. The controlled direction may include a direction determined by manual positioning of directional speaker towards an expected or desired location of a patient or a known receiving device location and/or may include a direction automatically determined using tracking systems for determining the location of a patient. A volume of the alarm conveyed by the directional sound waves may be configured to increase automatically from a quieter volume to a louder volume. At a step 708, optionally, a visual display of the alarm is presented using the directional alarm system in a manner to be in a line of sight of the patient. At a decision step 710, it is assessed if an alarm has been addressed within a specified time period. If the alarm has been addressed, then processing is ended for the iteration of the alarm notification handling process being described. If the alarm generated by the alarm generation component has not been addressed for a specified time, then processing proceed to a step 712, where a safety protocol is initiated that may include stopping emission of the directional sound waves and causing audible general broadcast of the alarm and/or other safety procedures.

Embodiments or implementations discussed herein may be combined with each other in appropriate combinations in connection with the system described herein. Additionally, in some instances, the order of steps in the flow diagrams, flowcharts and/or described flow processing may be modified, where appropriate. The system may further include a display and/or other computer components for providing a suitable interface with a user and/or with other computers. Aspects of the system described herein may be implemented or controlled using software, hardware, a combination of software and hardware and/or other computer-implemented or computer-controlled modules or devices having described features and performing described functions. Data exchange and/or signal transmissions to, from and between components of the system may be performed using wired or wireless communication. This communication may include use of one or more transmitter or receiver components that securely exchange information via a network, such as the Internet, and may include use of components of local area networks (LANs) or other smaller scale networks, such as Wi-Fi, Bluetooth or other short range transmission protocols, and/or components of wide area networks (WANs) or other larger scale networks, such as mobile telecommunication networks.

Software implementations of aspects of the system described herein may include executable code that is stored in a computer-readable medium and executed by one or more processors. The computer-readable medium may include volatile memory and/or non-volatile memory, and may include, for example, a computer hard drive, ROM, RAM, flash memory, portable computer storage media, an SD card, a flash drive or other drive with, for example, a universal serial bus (USB) interface, and/or any other appropriate tangible or non-transitory computer-readable medium or computer memory on which executable code may be stored and executed by a processor. The system described herein may be used in connection with any appropriate operating system. The meanings of any method steps of the invention(s) described herein are intended to include any suitable method of causing one or more parties or entities to perform the steps unless a different meaning is expressly provided or otherwise clear from the context.

As used herein, an element or operation recited in the singular and preceded with the word "a" or "an" should be understood as not excluding plural elements or operations, unless such exclusion is explicitly recited. References to "one" embodiment or implementation of the present disclosure are not intended to be interpreted as excluding the existence of additional embodiments that also incorporate the recited features. Furthermore, a description or recitation in the general form of "at least one of [a], [b] or [c]," or equivalent thereof, should be generally construed to include [a] alone, [b] alone, [c] alone, or any combination of [a], [b] and [c].

Embodiments and implementations of the invention will be apparent to those skilled in the art from a consideration of the specification or practice of the invention disclosed herein. It is intended that the specification and examples be considered as exemplary only, with the true scope of the invention being indicated by the following claims.

## Claims

1. A home dialysis system (310) comprising:
a dialysis machine (102, 202, 302) having an alarm generation component;
a directional alarm system (350, 450, 550, 650) communicatively coupled to the dialysis machine, wherein the directional alarm system includes a directional speaker configured to generate directional sound waves (355, 555),
wherein, in response to generation of an alarm by the alarm generation component of the dialysis machine, the directional speaker is configured to emit the directional sound waves indicating the alarm in a controlled direction towards a patient (355).

2. The home dialysis system according to claim 1, wherein the directional alarm system further comprises an orientation mechanism configured to orient the emitting of the directional sound waves in the controlled direction directed towards the patient (355).

3. The home dialysis system according to claim 2, wherein the orientation mechanism is a manual mechanism.

4. The home dialysis system according to claim 2, wherein the orientation mechanism is an automated mechanism that includes a sensor configured and used to locate automatically the patient (355) for emitting the directional sound waves.

5. The home dialysis system according to any of the preceding claims, wherein, in response to the generation of the alarm, the alarm generation component is configured to perform alarm processing and to deactivate a speaker of the dialysis machine for a period of time while the directional alarm system is functioning.

6. The home dialysis system according to any of the preceding claims, wherein the directional alarm system further includes a display component (118, 195, 452) that is configured to provide visual display of an alarm.

7. The home dialysis system according to any of the preceding claims, wherein the directional alarm system includes a receiving device (560) disposed remotely from the dialysis machine that is configured to receive the directional sound waves and direct the directional sound waves (565) towards a fixed location.

8. The home dialysis system according to any of the preceding claims, wherein the directional alarm system is configured to be in wireless communication with the dialysis machine.

9. The home dialysis system according to any of the preceding claims, wherein, if the alarm generated by the alarm generation component is not addressed for a specified time, a safety protocol is initiated that includes stopping emission of the directional sound waves and the dialysis machine (102, 202, 302) is configured to audibly generally broadcast the alarm.

10. The home dialysis system according to any of the preceding claims, wherein the directional alarm system is configured to increase a volume of the alarm conveyed by the directional sound waves automatically from a quieter volume to a louder volume.

11. The home dialysis system according to any of the preceding claims, wherein:
the dialysis machine (102, 202, 302) includes software and hardware components configured to detect errors, anomalies, inconsistencies, exceeding of set thresholds in connection with a dialysis treatment; and
the alarm generation component is configured to process an alarm as a result of the detection, the alarm processing including the generation of the alarm.

12. A non-transitory computer readable medium storing executable code that, when executed by a processor, performs a method for handling notification of a dialysis alarm by a home dialysis system, the method comprising:
generating (702) an alarm at a dialysis machine (102, 202, 302) of the home dialysis system using an alarm generation component;
activating (704) a directional alarm system (350, 450, 550, 650) communicatively coupled to the dialysis machine, wherein the directional alarm system includes a directional speaker that generates directional sound waves (355, 555);
in response to the generation of an alarm by the alarm generation component of the dialysis machine, emitting (706) the directional sound waves indicating the alarm from the directional speaker in a controlled direction towards a patient (355).

13. The computer readable medium according to claim 12, wherein the method comprises, in response to the generation of the alarm, deactivating a speaker of the dialysis machine for a period of time while the directional alarm system is functioning.

14. The computer readable medium according to any of claims 12-13, wherein the method further comprises visually displaying the alarm using the directional alarm system.

15. The computer readable medium according to any of claims 12-14, wherein the method further comprises, if the alarm generated by the alarm generation component is not addressed for a specified time, initiating a safety protocol that includes stopping emission of the directional sound waves and causing audible general broadcast of the alarm.

16. The computer readable medium according to any of claims 12-15, wherein the method further comprises increasing, automatically from a quieter volume to a louder volume, a volume of the alarm conveyed by the directional sound waves.

## Patentansprüche

1. Heimdialysesystem (310), das Folgendes umfasst:
eine Dialysemaschine (102, 202, 302) mit einer Alarmerzeugungskomponente;
ein gerichtetes Alarmsystem (350, 450, 550, 650), das kommunikationstechnisch an die Dialysemaschine gekoppelt ist, wobei das gerichtete Alarmsystem einen gerichteten Lautsprecher enthält, der konfiguriert ist, gerichtete Schallwellen (355, 555) zu erzeugen,
wobei der gerichtete Lautsprecher konfiguriert ist, als Reaktion auf die Erzeugung eines Alarms durch die Alarmerzeugungskomponente der Dialysemaschine gerichtete Schallwellen, die den Alarm angeben, in einer gesteuerten Richtung in Richtung eines Patienten (355) auszusenden.

2. Heimdialysesystem nach Anspruch 1, wobei das gerichtete Alarmsystem ferner einen Ausrichtungsmechanismus umfasst, der konfiguriert ist, das Aussenden der gerichteten Schallwellen in der gesteuerten Richtung, die in Richtung des Patienten (355) gerichtet ist, auszurichten.

3. Heimdialysesystem nach Anspruch 2, wobei der Ausrichtungsmechanismus ein manueller Mechanismus ist.

4. Heimdialysesystem nach Anspruch 2, wobei der Ausrichtungsmechanismus ein automatisierter Mechanismus ist, der einen Sensor enthält, der konfiguriert ist und verwendet wird, um den Patienten (355) automatisch zu lokalisieren, um die gerichteten Schallwellen auszusenden.

5. Heimdialysesystem nach einem der vorhergehenden Ansprüche, wobei die Alarmerzeugungskomponente konfiguriert ist, als Reaktion auf die Erzeugung des Alarms eine Alarmverarbeitung auszuführen und einen Lautsprecher der Dialysemaschine für eine Zeitperiode zu deaktivieren, während das gerichtete Alarmsystem arbeitet.

6. Heimdialysesystem nach einem der vorhergehenden Ansprüche, wobei das gerichtete Alarmsystem ferner eine Anzeigekomponente (118, 195, 452) enthält, die konfiguriert ist, eine visuelle Anzeige eines Alarms zu liefern.

7. Heimdialysesystem nach einem der vorhergehenden Ansprüche, wobei das gerichtete Alarmsystem eine Empfangsvorrichtung (560) enthält, die entfernt von der Dialysemaschine angeordnet ist, die konfiguriert ist, die gerichteten Schallwellen zu empfangen und die gerichteten Schallwellen (565) in Richtung eines festgelegten Ortes zu richten.

8. Heimdialysesystem nach einem der vorhergehenden Ansprüche, wobei das gerichtete Alarmsystem konfiguriert ist, mit der Dialysemaschine in drahtloser Kommunikation zu sein.

9. Heimdialysesystem nach einem der vorhergehenden Ansprüche, wobei dann, wenn der durch die Alarmerzeugungskomponente erzeugte Alarm nicht auf einen spezifizierten Zeitpunkt gerichtet ist, ein Sicherheitsprotokoll gestartet wird, das enthält, ein Aussenden der gerichteten Schallwellen zu stoppen, und die Dialysemaschine (102, 202, 302) konfiguriert ist, den Alarm hörbar allgemein auszusenden.

10. Heimdialysesystem nach einem der vorhergehenden Ansprüche, wobei das gerichtete Alarmsystem konfiguriert ist, eine Lautstärke des durch die gerichteten Schallwellen übermittelten Alarms automatisch von einer geringeren Lautstärke zu einer größeren Lautstärke zu erhöhen.

11. Heimdialysesystem nach einem der vorhergehenden Ansprüche, wobei:
die Dialysemaschine (102, 202, 302) Software- und Hardware-Komponenten enthält, die konfiguriert sind, Fehler, Unregelmäßigkeiten, Ungereimtheiten, Überschreiten von eingestellten Schwellenwerten in Verbindung mit der Dialysebehandlung zu detektieren; und
die Alarmerzeugungskomponente konfiguriert ist, einen Alarm als ein Ergebnis der Detektion zu verarbeiten, wobei die Alarmverarbeitung die Erzeugung des Alarms enthält.

12. Nicht transitorisches computerlesbares Medium, das ausführbaren Code speichert, der dann, wenn er durch einen Prozessor ausgeführt wird, ein Verfahren zum Behandeln einer Meldung eines Dialysealarms durch ein Heimdialysesystem ausführt, wobei das Verfahren Folgendes umfasst:
Erzeugen (702) eines Alarms an einer Dialysemaschine (102, 202, 302) des Heimdialysesystems unter Verwendung einer Alarmerzeugungskomponente;
Aktivieren (704) eines gerichteten Alarmsystems (350, 450, 550, 650), das kommunikationstechnisch an die Dialysemaschine gekoppelt ist, wobei das gerichtete Alarmsystem einen gerichteten Lautsprecher enthält, der gerichtete Schallwellen (355, 555) erzeugt;
als Reaktion auf die Erzeugung eines Alarms durch die Alarmerzeugungskomponente der Dialysemaschine Aussenden (706) der gerichteten Schallwellen, die den Alarm angeben, von dem gerichteten Lautsprecher in einer gesteuerten Richtung in Richtung eines Patienten (355).

13. Computerlesbares Medium nach Anspruch 12, wobei das Verfahren umfasst, als Reaktion auf die Erzeugung des Alarms einen Lautsprecher der Dialysemaschine für eine Zeitperiode zu deaktivieren, während das gerichtete Alarmsystem arbeitet.

14. Computerlesbares Medium nach einem der Ansprüche 12-13, wobei das Verfahren ferner umfasst, den Alarm unter Verwendung des gerichteten Alarmsystems visuell anzuzeigen.

15. Computerlesbares Medium nach einem der Ansprüche 12-14, wobei das Verfahren ferner umfasst, dann, wenn der durch die Alarmerzeugungskomponente erzeugte Alarm nicht auf einen spezifizierten Zeitpunkt gerichtet ist, ein Sicherheitsprotokoll einzuleiten, das enthält, ein Aussenden der gerichteten Schallwellen zu stoppen, und ein hörbares allgemeines Aussenden des Alarms zu bewirken.

16. Computerlesbares Medium nach einem der Ansprüche 12-15, wobei das Verfahren ferner umfasst, eine Lautstärke des durch die gerichteten Schallwellen übermittelten Alarms automatisch von einer geringeren Lautstärke zu einer größeren Lautstärke zu erhöhen.

## Revendications

1. Système (310) de dialyse à domicile comprenant :
une machine de dialyse (102, 202, 302) dotée d'un composant de génération d'alarme ;
un système d'alarme directionnelle (350, 450, 550, 650) couplé en communication avec la machine de dialyse, le système d'alarme directionnelle comportant un haut-parleur directionnel configuré pour générer des ondes sonores directionnelles (355, 555),
en réponse à la génération d'une alarme par le composant de génération d'alarme de la machine de dialyse, le haut-parleur directionnel étant configuré pour émettre les ondes sonores directionnelles indiquant l'alarme dans une direction contrôlée vers un patient (355).

2. Système de dialyse à domicile selon la revendication 1, dans lequel le système d'alarme directionnelle comprend en outre un mécanisme d'orientation configuré pour orienter l'émission des ondes sonores directionnelles dans la direction contrôlée dirigée vers le patient (355).

3. Système de dialyse à domicile selon la revendication 2, dans lequel le mécanisme d'orientation est un mécanisme manuel.

4. Système de dialyse à domicile selon la revendication 2, dans lequel le mécanisme d'orientation est un mécanisme automatisé comportant un capteur configuré et utilisé pour localiser automatiquement le patient (355) en vue de l'émission des ondes sonores directionnelles.

5. Système de dialyse à domicile selon l'une quelconque des revendications précédentes, dans lequel, en réponse à la génération de l'alarme, le composant de génération d'alarme est configuré pour réaliser un traitement d'alarme et pour désactiver un haut-parleur de la machine de dialyse pendant une période de temps tandis que le système d'alarme directionnelle fonctionne.

6. Système de dialyse à domicile selon l'une quelconque des revendications précédentes, dans lequel le système d'alarme directionnelle comporte en outre un composant d'affichage (118, 195, 452) configuré pour fournir un affichage visuel d'une alarme.

7. Système de dialyse à domicile selon l'une quelconque des revendications précédentes, dans lequel le système d'alarme directionnelle comporte un dispositif de réception (560), disposé à distance de la machine de dialyse, configuré pour recevoir les ondes sonores directionnelles et diriger les ondes sonores directionnelles (565) vers un emplacement fixe.

8. Système de dialyse à domicile selon l'une quelconque des revendications précédentes, dans lequel le système d'alarme directionnelle est configuré pour être en communication sans fil avec la machine de dialyse.

9. Système de dialyse à domicile selon l'une quelconque des revendications précédentes, dans lequel, si l'alarme générée par le composant de génération d'alarme n'est pas prise en compte pendant une durée spécifiée, un protocole de sécurité est lancé, lequel comporte l'arrêt de l'émission des ondes sonores directionnelles, et la machine de dialyse (102, 202, 302) est configurée pour diffuser l'alarme de manière audible et générale.

10. Système de dialyse à domicile selon l'une quelconque des revendications précédentes, dans lequel le système d'alarme directionnelle est configuré pour augmenter automatiquement, d'un volume plus faible à un volume plus fort, un volume de l'alarme transmise par les ondes sonores directionnelles.

11. Système de dialyse à domicile selon l'une quelconque des revendications précédentes, dans lequel :
la machine de dialyse (102, 202, 302) comporte des composants logiciels et matériels configurés pour détecter des erreurs, anomalies, incohérences, dépassements de seuils définis en relation avec un traitement de dialyse ; et
le composant de génération d'alarme est configuré pour traiter une alarme à la suite de la détection, le traitement de l'alarme comportant la génération de l'alarme.

12. Support non transitoire lisible par ordinateur stockant un code exécutable qui, lorsqu'il est exécuté par un processeur, réalise un procédé de gestion de notification d'une alarme de dialyse par un système de dialyse à domicile, le procédé comprenant :
la génération (702) d'une alarme au niveau d'une machine de dialyse (102, 202, 302) du système de dialyse à domicile à l'aide d'un composant de génération d'alarme ; l'activation (704) d'un système d'alarme directionnelle (350, 450, 550, 650) couplé en communication avec la machine de dialyse, le système d'alarme directionnelle comportant un haut-parleur directionnel générant des ondes sonores directionnelles (355, 555) ;
en réponse à la génération d'une alarme par le composant de génération d'alarme de la machine de dialyse, l'émission (706) des ondes sonores directionnelles indiquant l'alarme depuis le haut-parleur directionnel dans une direction contrôlée vers un patient (355).

13. Support lisible par ordinateur selon la revendication 12, dans lequel le procédé comprend, en réponse à la génération de l'alarme, la désactivation d'un haut-parleur de la machine de dialyse pendant une période de temps tandis que le système d'alarme directionnelle fonctionne.

14. Support lisible par ordinateur selon l'une quelconque des revendications 12 à 13, dans lequel le procédé comprend en outre l'affichage visuel de l'alarme à l'aide du système d'alarme directionnelle.

15. Support lisible par ordinateur selon l'une quelconque des revendications 12 à 14, dans lequel le procédé comprend en outre, si l'alarme générée par le composant de génération d'alarme n'est pas prise en compte pendant une durée spécifiée, le lancement d'un protocole de sécurité comportant l'arrêt de l'émission des ondes sonores directionnelles et le déclenchement d'une diffusion générale audible de l'alarme.

16. Support lisible par ordinateur selon l'une quelconque des revendications 12 à 15, dans lequel le procédé comprend en outre l'augmentation automatique, d'un volume plus faible à un volume plus fort, d'un volume de l'alarme transmise par les ondes sonores directionnelles.
